# EUROPEAN PATENT APPLICATION

(11) **EP 4 394 009 A1**
(43) Date of publication of application: **03.07.2024**
(21) Application number: 22315355.2
(22) Date of filing: 27.12.2022
(51) Int. Cl.: C09D 133/02, C09D 183/04, A61F 13/00, C09J 183/04

(54) **SILICONE LAYER HAVING ABSORBENT AND DESORBENT PROPERTIES**

(71) Applicant: Advanced Silicone Coating, 69330 Pusignan (FR)
(72) Inventor: Guillermin, Mélanie, 38230 CHARVIEU (FR)
(74) Representative: Littolff, Denis

(57) **Abstract**

The invention relates to new silicone layers showing excellent properties when being adhered to a surface such as skin. The silicone layer can for example be used as a wound contact layer for the preparation of articles such as wound dressings which are used for promoting/supporting the process of the healing of a wound.

## Description

The invention relates to new silicone layers showing excellent properties when being adhered to a surface such as skin. The silicone layer can for example be used as a wound contact layer for the preparation of articles such as wound dressings which are used for promoting/supporting the process of the healing of a wound.

A wound can be regarded as the separation of the contiguity of tissues of the skin, wherein this can be combined with loss of substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step. A suitable wound dressing may help to provide a favorable environment for said healing process.

Such wound dressings have to be applied on a surface such as a wound or the skin surrounding said wound. For this purpose, it is essential that the wound dressing adheres well to said surface to prevent an undesired slipping or even removal of the wound dressing. Within the field of wound dressing, polysiloxanes are known to have several advantages such as their adhesive properties. In addition, they exhibit atraumatic properties and provide a pleasant skin feeling.

Polysiloxanes (herein referred to as silicones) are also known for their hydrophobicity. This hydrophobicity, however, might cause issues, for example when the patient is sweating. Also oozing of the wound might create problems. The absorption rate of the build-up exudate is significantly reduced by the hydrophobicity of the silicone. In short, polysiloxane cannot properly absorb the exudate or sweat, and the corresponding liquid stays trapped between the wound and the hydrophobic silicone. This might lead to an inhibited wound healing or even infections of the wound as well as maceration of the wound itself and the surrounding skin. In addition, due to the above-mentioned liquids, the adherence of the wound contact layer might be negatively affected, and the wound dressing may fall off, and the wound will not be protected anymore.

Patent EP 1 713 523 B1 by Coloplast relates to an adhesive composition comprising hydrophilic silicone elastomers and hydrophobic silicone elastomers and optionally water absorbent material such as carboxy methyl cellulose (CMC), acrylates, alginates, chitosans, polysaccharides and derivates or mixtures thereof, wherein the ratio between the hydrophilic silicone elastomers and the hydrophobic silicone elastomers is from 95:5 to 5:95.

Said composition, however, seems to be improvable with regard to its structure, its absorbent and desorbent properties, such as the moisture vapor transmission rate (MVTR), and its peeling properties, such as the strength of the peel force.

Hence, it was an object of the present invention to overcome the above drawbacks.

In particular, it was an object of the present invention to provide a layer which can increase the wearing comfort as well as the wearing time.

Further, a layer wound contact layer which remains in place and prevents maceration of the skin, even in the occurrence of liquids- such as sweat and exudate, should be provided.

A further object of the present invention was the provision of a layer having breathable characteristics which allow the absorption and evaporation of ooze, and which may be applied as a durable coating on a substrate such as a textile or a layer of a wound dressing wherein it may optionally act as an adhesive.

Even further, it was an object to provide a layer which leads to an acceleration of the healing process of a wound while the positive properties of the silicone are maintained. In particular, it was an object of the present invention to provide a layer which can be used to absorb and desorb liquids such as exudate and sweat while the atraumatic and adhesive properties of the silicone are maintained. In other words, a wound contact layer should be provided whose adsorption rate of ooze is increased while the strength of the peel force ensures continuance in the place to which the wound dressing has been applied.

Especially, it was an object to provide a layer which can for example be used in a wound dressing which is capable of ensuring a strength of the peel force that ensures continuance at the place the wound dressing has been applied to, wherein the contact layer shows an increased adsorption rate from the wound and preferably passes adsorbed liquid to further layers of a wound dressing and/or which can be prepared in a simple way.

### Summary of the invention

The present invention is suitable to solve the above-listed objectives by a layer comprising a silicone and specific particles of an alkaline salt of a polymer having carboxylate groups wherein the silicone layer may be an adhesive layer.

Thus, the subject of the present invention is a layer comprising a silicone matrix containing particles of an alkaline salt of a polymer having carboxylate groups, wherein said particles have an average particle size D₅₀ of 1 to 40 µm.

It has surprisingly been found that a silicone layer can be provided which exhibits good absorbent and desorbent properties, while the adhesive properties such as a good peel force are maintained.

### Figures

Figure .1 shows the rheology of silicones having a content of 0%w/w, 20%w/w and 30%w/w of modified sodium poly(meth)acrylate particles.
Figure 2 shows the water vapor transmission rate of silicones having different contents of modified sodium poly(meth)acrylate particles, wherein said modified sodium poly(meth)acrylate particles have a D₅₀ value of 3.73 µm and about 40 µm, respectively.
Figure 3 shows the water vapor transmission rate of silicones at a coating weight of 110 g/m² and 140 g/m², respectively and different contents of modified sodium poly(meth)acrylate particles.
Figure 4 shows the peel adhesion of silicones at a coating weight of 80 g/m² and different contents of modified sodium poly(meth)acrylate particles.
Figure 5 shows the peel strength between a substrate (PET liner) and silicones having different contents of modified sodium poly(meth)acrylate particles, wherein said modified sodium poly(meth)acrylate particles have a D₅₀ value of 3.73 µm.
Figure 6 shows the absorption/desorption capacity of layers having a content of 0%w/w and 25%w/w of modified sodium poly(meth)acrylate particles, wherein said modified sodium poly(meth)acrylate particles have a D₅₀ value of 3.73 µm.

### Detailed description

The definitions are relevant in connection with the embodiments of the present invention.

The meaning of the term "comprising" is to be interpreted as encompassing all the specifically mentioned features as well as optional, additional unspecified ones, whereas the term "consisting of" only includes those features as specified. Therefore, "comprising" includes as a limiting case the meaning of "consisting of".

In this application term "about" may be understood as +/- 5% of the corresponding value.

The term "% by weight" "% w/w" are understood to refer to the weight amount of the component relative to the total weight of the adhesive silicone layer, if not specified otherwise.

The meaning of the term "alkyl" is to be interpreted to encompass linear, branched and cyclic alkyl residues. Thus, for example an "alkyl having 1 to 3 carbon atoms" encompasses methyl, ethyl, propyl, isopropyl and cyclopropyl.

The meaning of the term "alkylene" is to be interpreted to encompass an alkyl which has at least one (unsaturated) double bond.

The meaning of "alkaline salt" refers to a salt, where the cation is an alkaline cation, preferably sodium or potassium, more preferably sodium.

In line with the present application silicone can be regarded as a component which forms a matrix or scaffold embedding the particles of the alkaline salt of a polymer having carboxylate groups.

Generally, any silicone used in the present field of the art can be used as silicone for forming a silicone matrix.

Silicone can be regarded as polymers which comprise an silicon-oxygen (···-Si-O-Si-O-Si-O-···) chain as backbone wherein two organic groups are attached to each silicon, e.g. (-R₂Si-O-SiR₂-), wherein residue R is an organic group. A simple example is poly(dimethylsiloxan).

In a preferred embodiment of the present invention the silicone chains are crosslinked, i.e. there are single bond(s) or linker(s) containing preferably not more than 15 atoms which link the silicone chains with each other.

In a preferred embodiment of the invention, the silicone can be obtained by reacting one or more alkenyl-terminated poly(organo)siloxane(s) with one or more of Si-H containing poly(organo)siloxane(s).

Alkenyl-terminated poly(organo)siloxane(s) have
iA) at least one terminal siloxyl unit represented by the Formula (R¹)₂(R²) SiO_{1/2}, wherein R¹, which can be the same or different, are linear or branched alkyl groups with 1 to 6 carbon atoms or phenyl optionally substituted with linear or branched alkyl groups with 1 to 3 carbon atoms and R² is an alkenyl group having 2 to 6 carbon atoms, and
iiA) siloxyl units represented by the Formula (R³)₂ SiO_{2/2}, wherein R³, which can be the same or different, are linear or branched alkyl groups with 1 to 6 carbon atoms or phenyl optionally substituted with linear or branched alkyl groups with 1 to 3 carbon atoms aryl units.

In a preferred embodiment, in the at least one terminal siloxyl unit represented by the Formula (R¹)₂(R²) SiO_{1/2}, R¹ is the same and a linear or branched alkyl group with 1 to 3 carbon atoms or phenyl, preferably a linear or branched alkyl group with 1 to 3 carbon atoms, more preferably methyl.

In a preferred embodiment, in the at least one terminal siloxyl unit represented by the Formula (R¹)₂(R²) SiO_{1/2}, R¹ is the same and a linear or branched alkyl group with 1 to 3 carbon atoms or phenyl, more preferably methyl and R² is vinyl.

In a preferred embodiment, the alkenyl-terminated poly(organo)siloxane(s) comprise(s) two terminal siloxyl units represented by the Formula (R¹)₂(R²) SiO_{1/2}, preferably two terminal siloxyl units, wherein R² is vinyl. Such alkenyl-terminated poly(organo)-siloxane(s) can also be also referred to as vinyl-terminated poly(organo)siloxane.

It is preferred that alkenyl-terminated poly(organo)siloxane(s) are substantially linear polymers.

In a preferred embodiment, the alkenyl-terminated poly(organo)siloxane(s) are a mixture of different alkenyl-terminated poly(organo)siloxane(s), preferably a mixture of two to five, more preferably two, different alkenyl-terminated poly(organo)siloxane(s).

It is preferred that the alkenyl content, preferably the vinyl content, in the alkenyl-terminated poly(organo)siloxane(s) is 0.01 to 1, preferably 0.015 to 0.8 mmol/g of in the alkenyl-terminated poly(organo)siloxane(s).

It is preferred that the alkenyl content, preferably the vinyl content, in the alkenyl-terminated poly(organo)siloxane(s) is 0.03 to 0.35%, preferably 0.05 to 0.30%. In a preferred embodiment, there are two vinyl-terminated poly(organo)siloxanes, one with a vinyl content of 0.03 to 0.15%, preferably 0.05 to 0.12% and another one with a vinyl content of 0.18 to 0.35%, preferably 0.22 to 0.30%.

It is preferred that the viscosity at 25°C in the alkenyl-terminated poly(organo)siloxane(s) is 500 to 80000 centipoise, preferably 750 to 75000 centipoise. In a preferred embodiment, there are two vinyl-terminated poly(organo)siloxanes preferably vinyl-terminated poly(organo)siloxanes, one with a viscosity of 500 to 2000, preferably 750 to 1500 centipoise, and another one with a viscosity of 40000 to 8000, preferably 50000 to 75000 centipoise.

Si-H containing poly(organo)siloxane(s) have
iB) at least one terminal siloxyl unit represented by the Formula (H)_{q}(R⁴)ᵣSiO_{1/2}, wherein R⁴, which can be the same or different, are linear or branched alkyl groups with 1 to 6 carbon atoms or phenyl optionally substituted with linear or branched alkyl groups with 1 to 3 carbon atoms, q is selected from 1, 2 or 3 and q+r is equal to 3, and
iiB) siloxyl units represented by the Formula (H)ₛ(R⁵)ₜSiO_{2/2}, wherein R⁵, which can be the same or different, are linear or branched alkyl groups with 1 to 6 carbon atoms or phenyl optionally substituted with linear or branched alkyl groups with 1 to 3 carbon atoms aryl units, t is 1 or 2 and s+t is equal to 2.

In a preferred embodiment, in the at least one terminal siloxyl unit represented by the formula (H)_{q}(R⁴)ᵣSiO_{1/2}, R⁴ is the same and a linear or branched alkyl group with 1 to 3 carbon atoms or phenyl, preferably a linear or branched alkyl group with 1 to 3 carbon atoms, more preferably methyl.

It is further preferred that q is 1 or 2.

In a preferred embodiment in the siloxyl units represented by the formula (H)ₛ(R⁵)ₜSiO_{2/2}, wherein R⁵ is the same is a linear or branched alkyl groups with 1 to 3 carbon atoms or phenyl, preferably a linear or branched alkyl groups with 1 to 3 carbon atoms, more preferably methyl.

It is preferred that t is 2.

In a preferred embodiment, the Si-H containing poly(organo) siloxane(s) are a mixture of different Si-H containing poly(organo) siloxane(s), preferably a mixture of two to five, more preferably two, different Si-H containing poly(organo) siloxane(s).

It is preferred that the Si-group content in Si-H containing poly(organo) siloxane(s) is 0.5 to 20, more preferred 0.8 to 18, in particular 1.1 to 16 mmol/g of the Si-H containing poly(organo) siloxane(s).

It is preferred that the Si-group content in Si-H containing poly(organo) siloxane(s) is 3.5 to 6.5, preferably 4.0 to 60%.

It is preferred that the viscosity at 25°C, in the Si-H containing poly(organo) siloxane(s) is 5 to 500 centipoise, preferably 6 to 400 centipoise. In a preferred embodiment, there two Si-H containing poly(organo) siloxane(s), one with a viscosity of 5 to 25 centipoise and another one with a viscosity of 150 to 400 centipoise.

In a preferred embodiment of the invention, in the reaction of the alkenyl-terminated poly(organo)siloxane(s) and the Si-H containing poly(organo)siloxane(s), the molar ratio between the alkenyl-terminated poly(organo)siloxane(s) and the Si-H containing poly(organo)siloxane(s) can be between 1 : 6.5 and 1 : 0.2, preferably between 1 : 5.5 and 1 : 0.4, in particular between 1: 4.5 and 1 : 0.6.

In another preferred embodiment of the invention, the reaction of the alkenyl-terminated poly(organo)siloxane(s) and the Si-H containing poly(organo)siloxane(s) can be carried out in the presence of a catalyst. Generally, any catalyst suitable to carry out a hydrosilylation reaction can be used. Preferred are platinum-based catalysts. Non-limiting include platinum black, chloroplatinic acid, chloroplatinic acid modified with alcohol, oelfin, aldehyde, preferably platinum acetyl acetonate and platinum alcolates. Platinum can preferably be present in the corresponding catalyst in a content of between 5 to 10000 ppm. Further, if present, the catalyst can be used in an amount of between 0.001 to 0.5 by weight based on the weight of the sum of terminated poly(organo)siloxane(s) and the Si-H containing poly(organo)siloxane(s).

In a preferred embodiment, poly(organo)siloxane(s) not effecting the reaction of the alkenyl-terminated poly(organo)siloxane(s) and the Si-H containing poly(organo)siloxane(s) can be present during the reaction of the latter components. Non-limiting examples of such poly(organo)siloxane(s) include poly(dimetylsiloxane) and alpha,omega-trimethylsiloxyl-poly(dimetylsiloxane).

Further, inhibitors suitable to avoid an undesired start of the reaction, to control the reaction and amount of crosslinking and/or to terminate the reaction may be present. Such substances are known in the art. Non-limiting examples are tetramethyl vinyl tetrasiloxane, pyridine, phosphines and organic phosphines, unsaturated amides, alkyl maleates, preferably with an alkyl of 1 to 6 carbon atoms, acetylenic alcohols such as 1-ethynylclyclohexan-1-ol and generally substances bearing at least one alkenyl group. This substance can preferably be present in an amount of between 90 to 1100 ppm based on the weight of the sum of the alkenyl-terminated poly(organo)siloxane(s) and the Si-H containing poly(organo)siloxane(s).

In a preferred embodiment of the invention, the silicone consists of a matrix of hydrophobic silicone(s). Hydrophobic silicone(s) can be regarded as silicone having terminal polar groups such as hydroxy, amino and/or carboxy groups in an amount of less than 5%, preferably less than 3%, more preferably less than 1%, in particular less than 0.5%, based on the total number of end groups.

Otherwise said hydrophobic silicone(s) can be regarded as silicone having terminal non-polar groups such as alkylene, ether and unsubstituted phenyl groups in an amount of more than 95%, preferably more than 97%, more preferably more than 99%, in particular more than 99.5 %, based on the total number of end groups. The amount of polar and non-polar groups can be for example be determined via NMR with integral evaluation.

The adhesive silicone layer comprises particles of an alkaline salt of a polymer having carboxylate groups, wherein said particles have an average particle size D₅₀ of 1 to 40 µm. Generally, any polymer comprising carboxylate groups and having an average particle size D₅₀ of 1 to 40 µm can be used.

A polymer comprising carboxylate groups can be obtained for example by homopolymerizing a monomer having a carboxylate group such as acrylic acid (H₂C=CH(COOH)), methacrylic acid (H₂C=CCH₃(COOH)), maleic acid, itaconic acid and vinylpropionic acid or copolymerizing the before-mentioned monomers with further monomers. Suitable further monomers are known in the art. Non-limiting examples are vinyl compounds such as N-vinylpyrrolidon; vinyl esters such as vinyl formate and vinyl acetate; vinyl ethers such as methyl vinyl ether; vinyl ketones such as methyl vinyl ketone and ethyl vinyl ketone; vinyl halide compounds such as vinyl chloride and vinyl bromide, alkyl (meth)acrylate with alkyl having 1 to 6 atoms such as methyl acrylate, ethyl acrylate, butyl acrylate, methyl methacrylate, ethyl methacrylate, hexyl methacrylate, optionally substituted styrene compounds such as styrene and methyl styrene, vinylidene monomers such as vinylidene chloride, acrylamide and alkyl substituted compounds thereof with alkyl having 1 to 6 carbon atoms, acid compounds, salts and anhydrides thereof having a polymerizable double bond such as vinyl sulfonic acid, allyl sulfonic acid, methallyl sulfonic acid, styrene sulfonic acid, 2-acrylamido-2-methylpropane sulfonic acid. The corresponding polymer can be subsequently transferred to a salt by methods known in the art.

Alternatively, a polymer comprising carboxylate groups can be obtained for example by homopolymerizing a monomer having groups that can be further reacted to a carboxylate group such as acrylic nitril, alkyl (meth)acrylates and acrylamide and alkyl substituted compounds thereof with alkyl having 1 to 6 carbon atoms, acid compounds or copolymerizing before-mentioned monomers with further monomers. Suitable further monomers are known in the art. Non-limiting examples are vinyl compounds such as N-vinylpyrrolidon; vinyl esters such as vinyl formate and vinyl acetate; vinyl ethers such as methyl vinyl ether; vinyl ketones such as methyl vinyl ketone and ethyl vinyl ketone; vinyl halide compounds such as vinyl chloride and vinyl bromide, optionally substituted styrene compounds such as styrene and methyl styrene vinylidene monomers such as vinylidene chloride, salts and anhydrides thereof having a polymerizable double bond such as vinyl sulfonic acid, allyl sulfonic acid, methallyl sulfonic acid, styrene sulfonic acid, 2-acrylamido-2-methylpropane sulfonic acid. The corresponding polymer can be subsequently transferred to a polymer having carboxy groups and the salt thereof by methods known in the art.

Is it preferred that the polymer having carboxylate groups is a poly(meth)acrylate polymer or a poly(meth)acrylate copolymer. A polyacrylate polymer can be regarded as a polymer having repeating units of the formula -CH₂-CH(COO^{θ})-, while a polymethacrylate polymer can be regarded as a polymer having repeating units of the formula -CHCH₃-CH(COO^{θ})-. The corresponding copolymers thereof can further comprise units from the suitable monomer compounds as described above.

Further, the polymer having carboxylate groups is preferably crosslinked. As far as crosslinking is concerned, the same applies as described above. Preferably crosslinking is achieved by reacting a part of the carboxy groups or a part of the groups which can be further reacted to a carboxy group with guanidine or salts thereof, optionally substituted diamine such as ethylenediamine and methyl ethylenediamine or optionally substituted hydrazine or salts thereof.

Further, it is preferred that the polymer having carboxylate groups bears alkaline salt carboxylate groups in an amount of 1 to 15 meq/g, preferably 2 to 12 meq/g, more preferably 3 to 10 meq/g based on the weight of the polymer having carboxylate groups.

The particles of an alkaline salt of a polymer having carboxylate groups have an average particle size D₅₀ of 1 to 40 µm, preferably 2 to 35 µm, more preferably 3 to 30 µm

The expression "average particle size" can relate in the context of this invention to the D₅₀ value of the average particle size. The average particle size, which is also referred to as the D₅₀ value of the integral volume distribution, is defined in the context of this invention as the particle size at which 50% by volume of the particles have a smaller size than the size which corresponds to the D₅₀ value. Similarly, 50% by volume of the particles have a larger size than the D₅₀ value.
In a preferred embodiment of the invention, the particles of an alkaline salt of a polymer having carboxylate groups have a particle size (D₉₀) of 3 to 100 µm, preferably 5to 90 µm, more preferably 6 to 80 µm. The D₉₀ value of the integral volume distribution is defined in the context of this invention as the particle size at which 90% by volume of the particles have a smaller size than the size which corresponds to the D₉₀ value. Similarly, 10% by volume of the particles have a larger size than the D₉₀ value.

The particle sizes D₅₀ as well as D₉₀ are determined in accordance with ISO 13320:2020. The particle sizes D₅₀ as well as D₉₀ are determined by using a Mastersizer 3000 laser particle size analyzer from Malvern Instruments. With a Hydro LV liquid analysis module and Aero S dry analysis module. The evaluation is performed according to the Mie Theory.

In a preferred embodiment of the invention, the particles of an alkaline salt of a polymer having carboxylate groups have specific surface of 0.5 to 40 m²/g, preferably 1 to 30 m²/g, more preferably 2 to 30 m²/g, in particular 3 to 15 m²/g. With regard to the determination of the specific surface, it is referred to European Pharmacopoeia 2.9.26 ("Specific surface by gas adsorption"). The measurements of the specific surface according to the BET-method is carried out in accordance with ISO 9277:2010 using a 5.0 quality nitrogen. The determination can be carried out with a Gemini VII specific surface analyser from Micromeritics, a Smart VacPrep degaser from Micromeritics and a CRios-55 freeze dryer from Cyrotec.

In a preferred embodiment of the invention, the particles of an alkaline salt of a polymer having carboxylate groups have a porosity of 10 to 75%, preferably 20 to 70%, in particular 25 to 65%. The porosity is determined by mercury inclusion porosimetry in accordance with ISO 15901-1 9277: 2010. The determination can be carried out with an Autopore IV.

In a preferred embodiment of the invention, the particles of an alkaline salt of a polymer having carboxylate groups have a total pore area of 1 to 50 m²/g, preferably 5 to 40 m²/g, in particular 10 to 30 m²/g. The porosity is determined by mercury inclusion porosimetry in accordance with ISO 15901-1 9277: 2010. The determination can be carried out with an Autopore IV.

In a preferred embodiment of the invention the adhesive silicone layer may comprise one or more further substances, such as substances having an antimicrobial effect. Such substances are known in the art. Non-limiting examples include biguanide and derivatives thereof such as chlorhexidine, polyethylene biguanide (PEB), polytetramethylene biguanide (PTMB) or polyethylenhexamethylene biguanide (PEHMB); polygunnidine such as polyhexamethylene guanidine (PHMG), *N-*octyl-1-[10-(4-Octyliminopyridin-1-yl)decyl]pyridin-4-imine (octenidine), quaternary ammonium compounds such as benzalkonium chloride or cetylpyridinium chloride; triazine such as 1-(3-chloroallyl)-3,5,7-triaza-1-azonia-adamantan chloride, and taurolidine.

In a preferred embodiment the adhesive silicone layer has a thickness of from 20 µm to 225 µm, more preferably from 30 µm to 215 µm, in particular from 40 µm to 200 µm. Preferably the adhesive silicone layer has a thickness of about 45 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm or about 1.90 µm.

In a preferred embodiment of the invention, the particles of an alkaline salt of a polymer having carboxylate groups are present in an amount of 5 to 50% by weight, preferably 10 to 40% by weight, in particular 15 to 30 10 to 40% by weight based on the adhesive silicone layer.

It is preferred that the silicone adhesive layer has a water vapor transmission rate (WVTR) of 1000 to 25000 g/m²/24h determined according to DIN EN 13726, upright. In a preferred embodiment, the silicone adhesive layer has a water vapor transmission rate of about 2000 g/m²/24h, about 3000 g/m²/24h, about 4000 g/m²/24h, about 5000 g/m²/24h, about 6000 g/m²/24h, about 7000 g/m²/24h, about 8000 g/m²/24h, about 9000g/m²/24h, about 10000 g/m²/24h, about 11000 g/m²/24h, about 12000 g/m²/24h, about 13000 g/m²/24h, about 14000 g/m²/24h, about 15000 g/m²/24h, about 16000 g/m²/24h, about 17000 g/m²/24h, about 18000 g/m²/24h, about 19000 g/m²/24h, about 20000 g/m²/24h, about 21000 g/m²/24h, about 22000 g/m²/24h, about 23000 g/m²/24h, about 24000 g/m²/24h or about 25000 g/m²/24.

A further subject of the present invention is a method for preparing the adhesive silicone layer according to the present invention comprising the steps of:
(i) providing alkenyl-terminated poly(organo)siloxane(s)
(ii) Si-H poly(organo)containing siloxane(s),
(iii) adding the particles of an alkaline salt of a polymer having carboxylate groups to a blend of the component(s) of step (i) and the component(s) from step (ii),
(iv) coating the mixture from step (iii) on a substrate,
(v) subjecting the resulting mixture from step (iii) to a reaction.

As far as components are concerned, substantially the same applies as described above.

Step (i) includes the provision of the alkenyl-terminated poly(organo)siloxane(s). In alternative embodiments just a part of the alkenyl-terminated poly(organo)siloxane(s) is provided. It is preferred that a mixture of alkenyl-terminated poly(organo)siloxane(s) is used. If present, the alkenyl-terminated poly(organo)siloxane(s) can preferably comprise a catalyst.

Step (ii) includes the provision of Si-H containing poly(organo)siloxane(s) as component B. It is preferred that a mixture of Si-H containing siloxane(s) is used. In an alternative embodiment, if just a part of the alkenyl-terminated poly(organo)siloxane(s) in step (i) is provided, step (ii) can include the addition of the remaining part to the Si-H containing siloxane(s). Further, if present, the component(s) of step (ii) can preferably include an inhibitor.

Step (iii) includes the addition of the particles of an alkaline salt of a polymer having carboxylate groups to a blend of the component(s) from step (i) and the component(s) from step (ii).

"Blending" is understood in the context of the present invention as meaning a process of combining substances with the aim of achieving a substantially homogeneous distribution of different substances by the action of mechanical forces. Blending for the purposes of the invention is performed in conventional mixing devices such as tubular blenders, roll mixers, shaking mixers, free-fall mixers, shear mixers or intensive mixers. A tubular blender is preferably used.

The time for blending according to step (ii) may take for example 0.5 minutes to 1 hour, preferably 2 minutes to 50 minutes, in particular 5 minutes to 30 minutes.

The addition of the particles of an alkaline salt of a polymer having carboxylate groups can be done before, while or after blending the component(s) from step (i) and the component(s) from step (ii). In the latter case, it is preferred that the whole mixture is blended again.

Unlike the particles of an alkaline salt of a polymer having carboxylate groups, the optional one or more further substances such as substances having an antimicrobial effect and the optional poly(organo)siloxane(s) not effecting the reaction of the alkenyl-terminated poly(organo)siloxane(s) and the Si-H containing poly(organo)siloxane(s) can be added before, while or after blending the component(s) from step (i) and the component(s) from step (ii).

Step (iv) includes coating the mixture from step (iii) on a substrate. Preferably, the mixture from step (iii) can be coated with a coater on a substrate. Non-limiting examples for a material of a substrate are inert materials/polymers such as polyethylene-based materials, polypropylene-based materials, polyester-based materials, such as materials from polyethylene terephthalate (PET), polyamide-based materials, polyvinylchloride-based materials, polyacrylate-based materials, polymethacrylate-based materials, polyurethane-based materials, such as materials from polyester urethane or polyether urethane, silicone-based materials and mixtures thereof.

Further, the substrate can have any form as long as the mixture from step (iii)layer can be coated on one side of the substrate. Preferably the substrate is a film. The substrate can be regarded as a carrier of the adhesive silicone layer from which, if desired, it can be removed to be applied elsewhere.

The mixture from step (iii) can be coated on a substrate in an amount of 10 to 500 g/m², preferably 25 to 400 g/m2, more preferably 50 to 250 g/m², in particular 10 to 100 g/m²

In an alternative preferred embodiment, the mixture from step (iii) can be coated on a substrate in an amount of 0.5 to 25 g/m², preferably 0.75 to 20m², in particular 1 to 10 g/m².

Step (v) includes subjecting the mixture resulting from step (iii) to a reaction. By the reaction, the components are cured to form a silicone matrix comprising particles of an alkaline salt of a polymer having carboxylate groups.

Is it preferred that this step is carried out under blending conditions as described above.

In addition, subjecting the mixture resulting from step (iii) to a reaction preferably is carried out at elevated temperature. The term `at elevated temperature' as used herein refers to a temperature which is between 23°C and 200°C. 23°C and room temperature can be used interchangeably.

Alternatively, if an inhibitor is used, the term elevated temperature as used herein refers to a temperature which is between 23°C and 20°C above the boiling point of the inhibitor. Thus, the inhibitor is vaporized and the mixture resulting from step (iii) start to cure.

In a preferred embodiment of the invention the reaction in step (v) can preferably be carried out at a temperature between 60°C and 200°C, preferably between 80°C and 180°C, in particular between 90°C and 170°C.

In a preferred embodiment, the reaction in step (v) can preferably be carried out at a temperature of about 60°C, about 70°C, about 80°C, about 90°C, about 100°C, about 110°C, about 120°C, about 130°C, about 140°C, about 150°C, about 160°C, about 170°C, about 180°C, about 190°C or about 200°C

The time for subjecting the mixture resulting from step (iii) to a reaction, preferably being carried out at elevated temperature, may take for example 0.5 minutes to 2 hours, preferably 1 minutes to 30 minutes, more preferably 2 minutes to 15 minutes, in particular about 5 minutes.

A further subject of the present invention is the silicone layer obtainable by the method according to the present invention. The silicone layer may be an adhesive silicone layer.

According to one embodiment the adhesive silicone layer has definite areas of different adhesive strength or peel strength. Preferably, the adhesive silicone layer has two areas of different adhesive strength or peel strength.

A further subject of the present invention is a wound dressing comprising the adhesive silicone layer according to the invention and a further layer comprising absorbent material. An absorbent material can be referred to as a material capable of receiving and subsequently retaining a liquid such as water or exudate. Absorbent materials are known in the art. Non-limiting examples include compounds based on natural polymeric material such as starch, dextran, agarose, pectin, alginate, chitosan, hyaluronic acid, gellan, polypeptide and cellulose, wherein these natural polymeric materials can be further processed, for example by chemical derivation such as the formation of esters and ethers or pharmaceutically acceptable salts and compounds based on synthetic polymeric material such as polyvinyl alcohol, polyalkylene oxide, poly(meth)acrylate, poly(eth)acrylate, poly alkyl(meth)acrylate, poly alkyl(meth)acrylate, vinyl polymer, polycaprolactam and polycaprolactone, polyurethane, polyurea-based and polyurethane-polyurea-copolymers. In a preferred embodiment the absorbent material is a superabsorbent material based on a copolymer of acrylic acid and acrylamide or a foam, preferably a foam based on polyurethane, polyurea-based and polyurethane-polyurea-copolymers. It is preferred that the layer comprising absorbent materials has also desorption properties, i.e. that said layer may desorb liquids such as water.

In a preferred embodiment of the invention, the adhesive silicone layer is the wound contact layer. A wound contact layer can be regarded as the layer being directly in contact with the wound. It is further preferred that the further layer comprising an absorbent material is arranged directly in contact with the adhesive silicone layer according to the invention. Such an arrangement can for example have the advantage that exudate absorbed from the adhesive silicone layer on the wound side can be desorbed from the adhesive silicone layer on the side opposite to the wound and subsequently absorbed by the further layer comprising absorbing material, while liquids such as water can be absorbed/desorbed the other way. It is further preferred that the further layer comprising absorbent material such as a foam can preferably have a surface being smaller than the surface of the present adhesive silicone layer. It is further preferred that the further layer of absorbent material is arranged on or adhered to the centre of the present adhesive silicone layer such that the four rims of the adhesive silicone layer are not covered by the further layer comprising absorbent material and which may be substantially of the same area size thereby forming a continuous margin around the center. Wound dressings having such a continuous margin are defined as island dressings. Such an arrangement enables the adhesion of another layer such as a backing layer to the side opposite to the wound contact layer such that the top surface of the wound dressing is covered by the backing layer.

### Exemplary section

### 1. Preparation of a silicone layer according to the description

A mixture of 95 g vinyl-terminated poly(organo)siloxane having a vinyl content of 0.06 to 0.1% and a viscosity between 50000 to 70000 centipoise, 5 g vinyl-terminated poly(organo)siloxane having a vinyl content of 0.22 to 0.28 % and a viscosity between 800 to 12000 centipoise and a 0.005g platinum catalyst (platinum acetyl acetonate) are provided as Part A.

Further, a mixture of 93.3 g vinyl-terminated poly(organo)siloxane having a vinyl content of 0.06 to 0.1% and a viscosity between 50000 to 70000 centipoise, 4.9 g vinyl-terminated poly(organo)siloxane having a vinyl content of 0.22 to 0.28% and a viscosity between 800 to 12000 centipoise, 1,7 g Si-H containing poly(organo)siloxane having a Si-H content of 4.5 to 5.0% and a viscosity of 250 to 350 centipoise and 0,1 g Si-H containing poly(organo)siloxane having a Si-H content of 5.2 to 5.8% and a viscosity of 7.0 to 20350 centipoise and 0.8g of an inhibitor (1-ethynylclyclohexan-1-ol) are provided as Part B.

Furthermore, 0 g, 10 g, 20 g, 30 g, 40 g, 50 g, 60 g, 70 g and 80 g (corresponding to 0, 5, 10, 15, 20, 25, 30, 35 and 40% by weight based on the sum of Part A and Part B) of the particles of a sodium salt of a modified poly(meth)acrylate, wherein the particles have an average particle diameter D₅₀ of 1 to 40 µm were dried in an oven at 160°C for one hour as the corresponding Part C.

Parts A, B and C are blended to obtain mixtures M0 to M8 as shown in Table 1.

**Table 1:**

| | M0 | M1 | M2 | M3 | M4 | M5 | M6 | M7 | M8 |
|---|---|---|---|---|---|---|---|---|---|
| Part A [g] | 100 | 100 | 100 | 100 | 100 | 100 | 100 | 100 | 100 |
| Part B [g] | 100 | 100 | 100 | 100 | 100 | 100 | 100 | 100 | 100 |
| Part C [g] | 0 | 10 | 20 | 30 | 40 | 50 | 60 | 70 | 80 |

Each mixture was applied via the blade of a lab coater on a non-woven substrate in an amount of 80 g/m², Subsequently, the silicone is cured in an oven at 140°C for 5 minutes to obtain the present silicone layer.

### 2. Properties of the present silicone layer

### 2.1 Rheology of the product

The rheology was determined with a Rheometer AR 1000 using a computer equipped with software for the control of the rheometer and the analysis of the results, wherein procedure was carried out as follows:
- placing a weighted sample of the silicone in the center of the tray of the rheometer,
- subjecting the mobile to go down and ensuring that the crushed silicone covers the entire surface of the mobile,
- removing excess of silicone, if necessary,
- starting measurement.

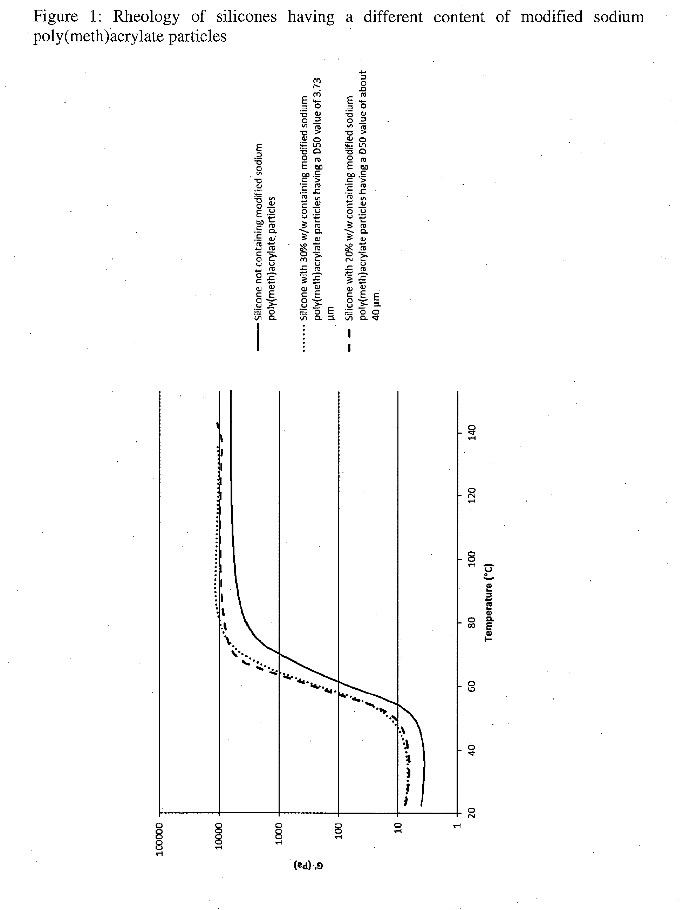

As can be seen from Figure 1, a silicone layer having a high content of modified sodium acrylate particles has substantially the same rheology as a silicone which does not contain said particles. Put differently, the incorporation of modified sodium acrylate particles to the mixture to be cured does not have a negative effect on the cured product.

### 2.2 Water vapor transmission rate

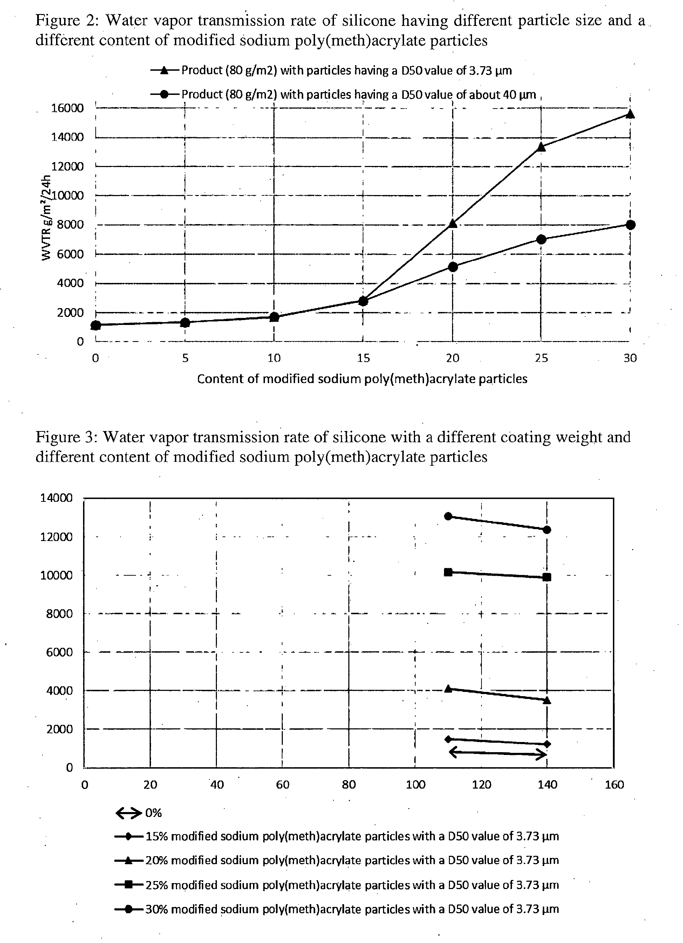

As described above, the water vapor transmission rate was determined according to DIN EN 13726, upright.
**A)** As can be seen from Figure 2, the present silicone layer exhibits an advantageously high water vapour transmission rate compared to a layer not comprising particles of a sodium salt of a modified poly(meth)acrylate. Taking this into account, it is assumed that due to this property the present layer enables the absorption of the liquids such as exudate or water on the one side, and the desorption thereof on the other side or vice versa.
**B)** Example 1 was carried out, wherein mixtures M3, M4; M5 and M6 were applied via the blade of a lab coater on a non-woven substrate in an amount of 110 g/m² and 140 g/m², respectively.

As can be seen form Figure 3, even at different thicknesses (resulting from the different amounts applied per m² (coating weight)) the present layer exhibits an excellent high water vapour transmission rate responsible for the high level of its breathability.

### 3. Peel adhesion

The peel adhesion was determined by a method based on FINAT n°1.

The peel adhesion of the present silicone layers having different contents of modified sodium poly(meth)acrylate particles was measured.

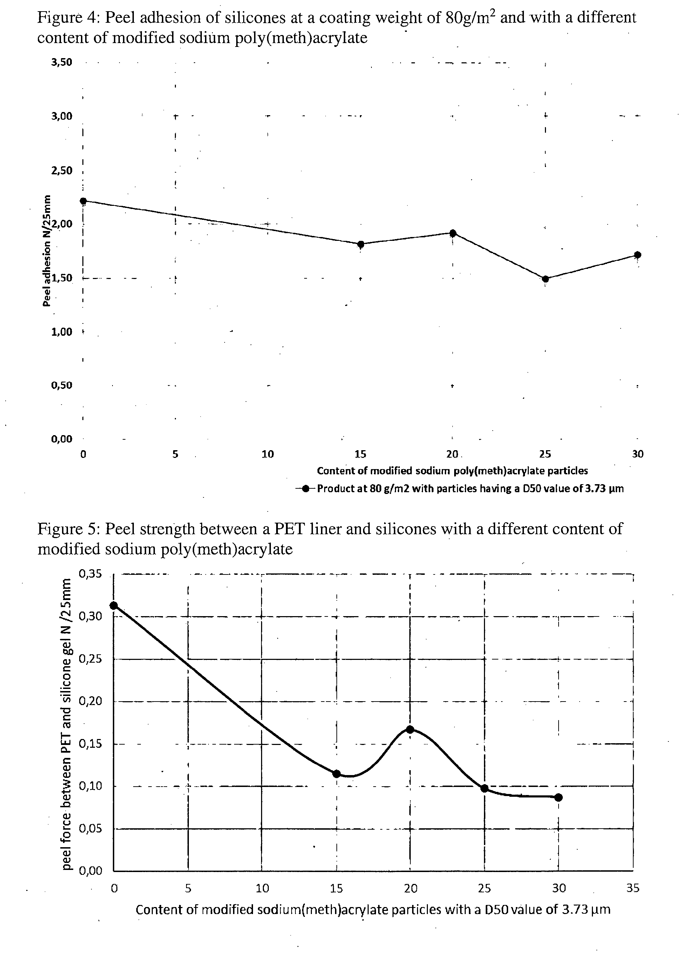

As can be seen from Figure 4, the present silicone layer has substantially the same peel adhesion as a silicone layer not containing sodium poly(meth)acrylate particles. Thus, the incorporation of sodium poly(meth)acrylate particles in a silicone layer does not have a negative impact on the peel adhesion compared with a silicone layer not containing modified sodium poly(meth)acrylate particles.

### 4. Peel strength

The peel force was determined by the following method:
- Preparing laminated test panels consist of two flexible adherends properly prepared and bonded together
- Cutting the bonded panels into 25-mm test specimens by a means that is not deleterious to the bond. The unbonded ends bent apart, perpendicular to the glue line, for clamping in the grips of the tension testing machine
- Conditioning specimens for 7 days at a relative humidity of 50 ± 2 % at 23 ± 1°C Clamping the bent, unbonded ends of the test specimen in the test grips of the tension testing machine.
- Applying the load at a constant head speed of 254 mm (10 in.)/min.
- Making an autographic recording of load versus distance peeled
- Determining the peel resistance over at least 130 mm
- Calculating the peel force

As can be seen from Figure 5, compared to a layer not containing modified sodium poly(meth)acrylate particles, the present silicone layer has an advantageous value with regard to the peel (release) strength of the PET liner such that an easier further processing of the layer for example in line with the preparation of a wound dressing is enabled .In other words the decrease of the force need to remove the PET liner of the silicone is advantageous for the processability of the material.

### 5. Absorption/Desorption capacity

The absorption and desorption capacity of a present silicone layer comprising 25% by weight of particles of modified sodium poly(meth)acrylate having an average particle diameter D₅₀ value of 3.73 µm and a silicone layer not containing such particles were compared, wherein the absorption and desorption capacities were determined according to the following method:
- round shaped samples each of 100 cm² were cut and weighted,
- the samples were put into water and weighted every 5 minutes,
- after having reached the maximum absorption capacity the samples were left in open air and weighted every five minutes,
- after having reached the maximum desorption, the examples were put again into water and the cycle of measurements started again.

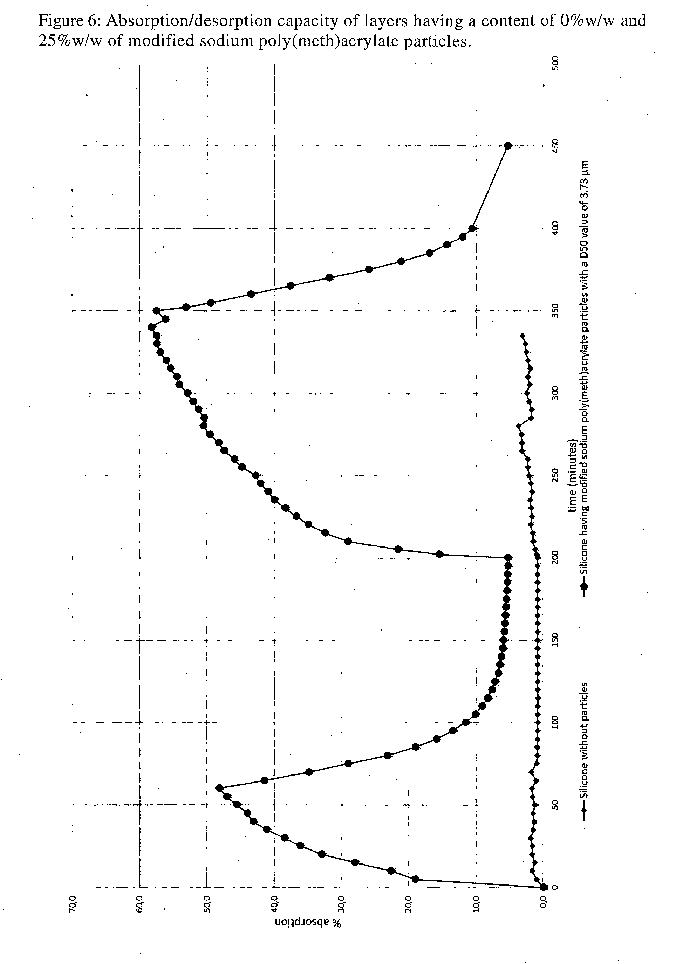

As can be seen from Figure 6, the present silicone layer can absorb and desorb humidity (water) several times, while a product not containing particles of modified sodium poly(meth) acrylate cannot absorb substantial amounts of humidity at all.

## Claims

1. Silicone layer comprising a silicone matrix containing particles of an alkaline salt of a polymer having carboxylate groups, wherein said particles have an average particle diameter D₅₀ of 1 to 40 µm.

2. Silicone layer according to claim 1, wherein the silicone matrix consists of hydrophobic silicone(s).

3. Silicone layer according to claim 1 or 2, wherein the polymer having carboxylate groups is a poly(meth)acrylate polymer.

4. Silicone layer according to any one of the preceding claims, wherein the particles have an average diameter of 4 to 30 µm.

5. Silicone layer according to any one of the preceding claims, wherein the particles of an alkaline salt of a polymer having carboxylate groups is present in an amount of 5 to 50% by weight based on the silicone layer.

6. Silicone layer according to any one of the preceding claims, wherein the particles have a porosity of 10 to 75%.

7. Silicone layer according to any one of the preceding claims, wherein the wound contact layer has a moisture vapor transmission rate (MVTR) of 1500 to 25000 g/m²/24h determined as described in the description.

8. Method for preparing a silicone layer according to any one of the preceding claims comprising the steps of:
(i) providing alkenyl-terminated polyorganosiloxane(s),
(ii) Si-H containing poly(organo)siloxane(s),
(iii) adding the particles of an alkaline salt of a polymer having carboxylate groups to a blend of the component(s) of step (i) and the component(s) from step (ii),
(iv) coating the mixture from step (iii) on a substrate, and
(v) subjecting the resulting mixture from step (iii).

9. Method according to claim 8, wherein component(s) from step (i) further comprise a catalyst and component(s) from step (ii) further comprise an inhibitor.

10. Method according to claim 8 or 9, wherein step (v) comprises heating the mixture to an elevated temperature.

11. Silicone layer obtainable by the method according to any one of claims 8 to 10.

12. Wound dressing comprising the silicone layer according to any one of claims 1 to 7 and 11 and a further layer comprising absorbent material.

13. Wound dressing according to claim 12, wherein the silicone layer is the wound contact layer.

14. Wound dressing according to claim 12 and/or claim 13, wherein the further layer comprising absorbent material is arranged directly on the silicone layer.
